# EUROPEAN PATENT SPECIFICATION

(11) **EP 0 462 631 B1**
(45) Date of publication and mention of the grant of the patent: **20.03.1996**
(21) Application number: 91114034.1
(22) Date of filing: 10.06.1986
(51) Int. Cl.: A61K 33/24, A61K 31/60, A61K 31/29, A61K 31/535, A61K 31/43, A61K 31/415

(54) **Methods and compositions for the treatment of gastrointestinal disorders**
Verfahren und Zusammensetzungen zur Behandlung von gastrointestinalen Störungen
Procédés et composés pour le traitement des désordres gastro-instestinaux

(30) Priority: 13.06.1985 US 744841
(43) Date of publication of application: 27.12.1991
(62) Divisional of application: 86304407.9
(73) Proprietor: Marshall, Barry James, Dr., Virginia 22936 (US)
(72) Inventor: Marshall, Barry James, Dr., Virginia 22936 (US)
(74) Representative: Brooks, Maxim Courtney

(56) References cited:
- FR-A- 1 332 522
- US-A- 3 395 212
- UNLISTED DRUGS, vol. 22, no. 11, November 1970, page 163, abstract b; "Aldefur"
- World Congress of Fastrenterology, August 26-31 1990, Sydney, Australia; "Helicobacter pylore: Casual atent in peptic ulcer disease?", p. 36-45
- Gastroenterology, 94, p. 33-40, 1988, Rauws et al
- Gastroenterology, 92(5), p. 1518, 1987, Marshall et al
- Gastroenterology, 92, nr. 5, part 2, p. 1324, 1987, Borody et al
- Med. J. Australia, 146, p. 450-451, 1984; Borody et al

## Description

This invention relates to kits-of-parts for the treatment of gastrointestinal disorders in humans and other animals.

Factors adversely affecting the function of the gastrointestinal system in humans are exceedingly varied in their nature. Such disorders may arise in the upper or lower gastrointestinal tracts or both. There is a broad range of causes of gastrointestinal disorders, including genetic, physiological, environmental, and psychogenic factors. Accordingly, the diagnosis and management of these disorders can be exceptionally difficult. A detailed discussion of gastrointestinal tract functions, disorders, causes, and treatments can be found in Spiro, Clinical Gastroenterology (3d. edition 1983).

Among the chronic disorders of the upper gastrointestinal tract are those which fall under the general categories of gastritis and peptic ulcer disease. (The upper gastrointestinal tract is generally defined as including the esophagus, the stomach, the duodenum, the jejunum, and ilium.) Gastritis is, by definition, typified by an inflammation of the stomach mucosa. In practice, though, the disorder is manifested by a broad range of poorly-defined, and heretofore inadequately treated, symptoms such as indigestion, "heart burn", dyspepsia and excessive eructation. A general discussion of gastritis appears in B. J. Marshall and J. R. Warren, "Unidentified Curved Bacilli in the Stomach of Patients with Gastritis and Peptic Ulceration", The Lancet, 1311-1315 (1984), and in R. Greenlaw, et al., "Gastroduodenitis, A Broader Concept of Peptic Ulcer Disease", 25 Digestive Diseases and Sciences 660-672 (1980).

Peptic ulcers are lesions of the gastrointestinal tract lining, characterized by loss of tissue due to the action of digestive acids and pepsin. It has been generally held that peptic ulcers are caused either by gastric hypersecretion, or (more often) by decreased resistance of the gastric lining to digestive acids and pepsin. The medical literature is replete with methods for treating ulcers, including modification of the diet, surgical removal of the lesions, and the use of drugs. Such drugs include: antacids, which serve to counteract excess gastric secretions; anticholinergics, which reduce acid secretion; H₂ antagonists, which also block the release of gastric acids; prostaglandins, which increase the resistance of the gastric lining to digestive fluids, and may also inhibit acid secretion; prokinetic agents, which enhance gastrointestinal tract motility; and compositions which form protective barriers over gastric lesions. Prescription and non-prescription drug therapies are generally described in Garnet, "Antacid Products", Handbook of Non-prescription Drugs, Chapter 3 (7th edition, 1982). One group of drugs which are thought to be effective due to coating of ulcer sites and forming protective barriers is the bismuth-containing drugs. See, for example, Koo, et al., "Selective Coating of Gastric Ulcers by Tripotassium Dicitrato Bismuthate in the Rat", 82 Gastroenterology 864-870 (1982).

Regardless of the particular drug composition used in treating gastrointestinal disorders, such as peptic ulcer disease, the treatment is often imprecise and incomplete. Actual "cures", i.e., successful treatment resulting in total remission of disease, are very often not effected. See, A. J. McLean, et al., "Cyto-protective Agents and Ulcer Relapse", 142 The Medical Journal of Australia, Special Supplement S25-S28 (1985). Furthermore, many conventional treatments may render subjects hypochlorhydric (i.e., with low levels of hydrochloric acid in the stomach) which may predispose them to other disorders, e.g., gastrointestinal infections, halitosis, and gastric carcinomas.

It has now been discovered that certain kits-of-parts comprising daily doses of bismuth and of antimicrobials, are effective for the treatment of gastrointestinal disorders. In particular, as compared to the art, these kits-of-parts cure, or afford lower relapse rates of, gastritis and peptic ulcer disease.

These kits-of-parts also afford other benefits in the treatment and management of subjects having gastrointestinal diseases, such as in not rendering treated subjects hypochlorhydric.

According to the invention, there is provided a kit-of-parts for use in the treatment of a human or other animal subject having an infectious gastrointestinal disorder, the kit-of-parts comprising from 3 to 56 daily dosages of a bismuth compound or bismuth-containing composition containing from 50 to 5000 mg of bismuth per dosage and from 1 to 21 daily dosages of an antimicrobial or antimicrobial-containing composition containing from 100 to 10,000 mg of antimicrobial per dosage and wherein the antimicrobial is selected from gentamicin, neomycin, kanamycin, streptomycin, erythromycin, clindamycin, rifampin, penicillin G, penicillin V, ampicillin, amoxycillin, bactracin, polymyxin, tetracycline, chlortetracycline, oxytetracycline, doxycyline, cephalexin, cephalothin, chloramphenicol, sulfonamides, nitrofurazone, nitrofurantoin, furozolidone, metronidazole, tinidazole, nimorazole, and mixtures thereof.

Specific compounds and compositions to be used in the kits-of-parts of the present invention must, accordingly, be pharmaceutically-acceptable. As used herein, such a "pharmaceutically-acceptable" component is one which is suitable for use with humans and/or animals without undue adverse side effects (such as toxicity, irritation, and allergic response) commensurate with a reasonable benefit/risk ratio. Further, as used herein, the term "safe and effective amount" refers to the quantity of a component which is sufficient to yield a desired therapeutic response without undue adverse effects (such as toxicity, irritation, or allergic response) commensurate with a reasonable benefit/risk ratio when used in the manner of this invention. The specific "safe and effective amount" will, obviously, vary with such factors as the particular condition that is being treated, the severity of the condition, the duration of the treatment, the physical condition of the patient, the nature of concurrent therapy (if any), and the specific formulations employed in the present invention.

As used herein, "gastrointestinal disorder" encompasses any disease or other disorder of the upper gastrointestinal tract of a human or lower animal. Such gastrointestinal disorders include, for example: disorders not manifested by presence of ulcerations in the gastric mucosa (herein "non-ulcerative gastrointestinal disorder"), including chronic or atrophic gastritis, non-ulcer dyspepsia, esophogeal reflux disease and gastric motility disorders; and "peptic ulcer disease", i.e., gastric, duodenal and jejunal ulcers. In particular, "gastrointestinal disorder" refers to such disorders of the upper gastrointestinal tract caused or mediated by bacteria, including campylobacter-like organisms (herein "CLO"), e.g., Campylobacter pyloridis. Such CLO include those described in J. R. Warren and B. J. Marshall, "Unidentified Curved Bacilli on Gastric Epithelium in Active Chronic Gastritis", The Lancet 1273-1275 (1983) and G. Kasper and N. Dickgiesser, "Isolation from Gastric Epithelium of Campylobacter-like Bacteria that are Distinct from 'Campylobacter Pyloridis'", The Lancet 111-112 (1985).

As used herein, "administering" refers to any method which, in sound medical practice, delivers the compounds or compositions used in this invention to the subject to be treated in such a manner so as to be effective in the treatment of the gastrointestinal disorder.

### Bismuth:

The kits-of-parts of this invention comprise a number of daily dosages suitable for administration of from 50 mg to 5000 mg of bismuth, per day, for from 3 to 56 days. (As used herein, the quantity of bismuth is by weight of elemental bismuth. Thus, the actual weight of a bismuth-containing compound will be greater.) Preferably, the daily dosages are from 500 mg to 1500 mg of bismuth. The number of daily dosages of bismuth will vary according to the specific gastrointestinal disorder to be treated. In general, though, non-ulcerative gastrointestinal disorders require 3 to 21 daily dosages of bismuth. Peptic ulcer disease requires from 14 to 56 daily dosages.

In the kits of this invention, the bismuth is preferably used as a pharmaceutically-acceptable salt. Such bismuth salts include, for example, bismuth aluminate, bismuth subcarbonate, bismuth subcitrate, bismuth citrate, tripotassium dicitrato bismuthate, bismuth subgalate, bismuth subnitrate, bismuth tartrate, bismuth subsalicylate, and mixtures thereof. Bismuth citrate, bismuth subcitrate, tripotassium dicitrato bismuthate, bismuth tartrate, bismuth subsalicylate, and mixtures thereof are preferred bismuth salts for use in this invention. The bismuth useful herein may be used alone, or in combination with other pharmaceutically-acceptable components, in a bismuth-containing composition. A variety of such compositions containing bismuth salts are commercially-available, including, for example, DeNol (RTM), containing tripotassium dicitrato bismuthate (sold by Gist-Brocades N.V.), Noralac, containing bismuth aluminate, alginic acid, and magnesium carbonate (manufactured by North American Pharmaceuticals), Roter (RTM) bismuth, containing bismuth subnitrate (sold by Roter Laboratories), Fensobar Polvo (RTM), containing bismuth subcarbonate among other materials (manufactured by USV Pharmaceutical Corporation), and Pepto-Bismol (RTM), containing bismuth subsalicylate (sold by The Procter & Gamble Company).

### Antimicrobial:

The kits of the present invention also include from 1 to 21 daily dosages of an antimicrobial containing from 100 mg to about 10,000 mg per dosage Preferably, the kit comprises from 1 to 14 daily dosages of antimicrobial. The specific dosage of antimicrobial to be administered, as well as the duration of antimicrobial treatment, are mutually dependent, and will also depend upon such factors as the specific antimicrobial used, the resistance pattern of the infecting organism to the antimicrobial used, the ability of the antimicrobial to reach minimum inhibitory concentrations at the site of the infection, the nature and extent of other infections (if any), the personal attributes of the subject, compliance with the treatment regimen, and the presence and severity of any side effects of the treatment.

A wide variety of antimicrobials are useful in this invention. As used herein, such "antimicrobials" refer to a naturally-occurring, synthetic or semi-synthetic antibiotic compound or composition, or mixture thereof, which is safe for human use as used in the processes of this invention, and is effective in killing or substantially inhibiting the growth of CLO when used in the processes of this invention. Suitable antibiotics can be generally classified by chemical composition, into the following principal groups: aminoglycosides selected from gentamicin, neomycin, kanamycin, and streptomycin; macrolides selected from erythromycin, clindamycin, and rifampin; penicillins selected from penicillin G, penicillin V, ampicillin and amoxycillin; polypeptides selected from bacitracin and polymyxin; tetracyclines selected from tetracycline, chlortetracycline, oxytetracycline, and doxycycline; cephalosporins selected from cephalexin and cephalothin; and the miscellaneous antibiotic chloramphenicol. These antibiotics can be generally said to function in one of four ways: inhibition of cell walls synthesis, alteration of cell wall permeability, inhibition of protein synthesis, or inhibition of nucleic acid synthesis.

Other antimicrobials useful herein are the sulfonamides; nitrofurans selected from nitrofurazone, nitrofurantoin, and furozolidone; and metronidazole, tinidazole, and nimorazole. Antimicrobials among those useful herein are described in the following publications; Remington's Pharmaceutical Sciences (15th edition 1975); F. H. Meyers, et al., Review of Medical Pharmacology (7th edition 1980); Gaddum's Pharmocology (8th edition 1978); and A. Goodman, A. G. Goodman and L. S. Gilman, The Pharmacological Basis of Therapeutics (6th edition 1980).

While any of these antimicrobials may be used, penicillin, erythromycin, doxycycline, metronidazole, tinidazole, amoxycillin, ampicillin, and nitrofurantoin are among the preferred antimicrobials for use in the present invention. Preferably, a sample of CLO is obtained from the stomach of the subject to be treated, as by biopsy, aspiration, or by other suitable method, and the organism cultured and tested for sensitivity to the various antimicrobials useful herein. Preferably such sensitivity testing is by determination of the relative minimum inhibitory concentrations of the antimicrobials using broth or plate dilution techniques. The antimicrobial found to be most effective against the cultured bacteria (i.e., effective at the lowest minimum inhibitory concentration) is then selected for use in this invention.

As stated above, the specific preferred quantity of antimicrobial, and duration of treatment will, in addition to other factors, depend upon the particular antimicrobial used and its pharmacology. In general, though, the tetracyclines are preferably administered at a level of from about 100 mg to about 2000 mg per day. Macrolides (such as erythromycin) are preferably administered at a level of from about 1000 mg to about 4000 mg per day. Penicillins are preferably administered at a level of from about 500 mg to about 3000 mg per day. The aminoglycosides (such as neomycin) are, preferably, administered at a level of from about 100 mg to about 8000 mg per day. Nitrofurans (such as nitrofurantoin) are administered preferably at levels of from about 100 mg to about 800 mg per day. Preferably, metronidazole is administered at a level of from about 500 to about 2000 mg per day.

The specific method of administering the antimicrobial, may depend upon such factors as the particular antimicrobial used, the site of infection, the amount of antimicrobial to be administered per day, the presence of any adverse side effects, and the interactions (if any) between the antimicrobial and the bismuth. Thus, the antimicrobials may be administered by single daily doses, or by administration in two, three, four, or more doses per day. One factor, in particular, is potential interaction between the antimicrobial and the bismuth administered under these processes. For example, the presence of bismuth is known to adversely affect the efficacy of the tetracyclines. See, for example, C.D. Ericsson, et. al., "Influence of Subsalicylate Bismuth on Absorption of Doxycycline" 247 J. of American Medical Assoc. 2266 (1982). Hence, it is preferred to adminster those antimicrobials that are subject to adverse bismuth interaction by methods that minimize such interactions, i.e., by minimizing the simultaneous presence of antimicrobial and bismuth in the stomach. Such methods include one or more of the following: staggered oral dosing of the bismuth and antimicrobial, through discrete administration of each compound or composition separated by at least (preferably) two hours between dosages; oral administration of the antimicrobial in an enterically coated form, i.e., coating of the antimicrobial which prevents dissolution of the antimicrobial in the stomach; use of processes wherein the step of administering bismuth is terminated prior to commencing the step of orally administering the antimicrobial; and administering the antimicrobial by a non-oral route, e.g., by intraveneous or intramuscular injection.

The compositions used in the kits of the present invention may contain optional components which affect the physical and therapeutic characteristics of the compositions. In particular, a variety of pharmaceutically-acceptable carriers and excipients may be included, depending upon the particular dosage form to be used. Various oral dosage forms can be used, including such solid forms as tablets, capsules, granules and bulk powders. Tablets can be compressed, tablet triturates, enteric-coated, sugar-coated, film-coated or multiple compressed, containing suitable binders, lubricants, diluents, disintegrating agents, coloring agents, flavoring agents, flow-inducing agents and melting agents. Liquid oral dosage forms include aqueous solutions, emulsions, suspensions, solutions and/or suspensions reconstituted from non-effervescent granules and effervescent preparations reconstituted from effervescent granules, containing suitable solvents, preservatives, emulsifying agents, suspending agents, diluents, sweeteners, melting agents, coloring, and flavoring agents.

Specific examples of pharmaceutically-acceptable carriers and excipients that may be used to formulate oral dosage forms are described in US-A-3,903,297, Robert, issued September 2, 1975. Techniques and compositions for making dosage forms useful herein are described in the following references; 7 Modern Pharmaceutics, Chapters 9 and 10 (Banker and Rhodes, editors, 1979); an Lieberman, et al., Pharmaceutical Dosage Forms: Tablets (1981); and Ansel, Introduction to Pharmaceutical Dosage Forms (2d edition, 1976).

A preferred method of use of the kits of the present invention involves a delay, preferably of from 1 to 21 days, more preferably from 1 to 10 days, more preferably from 1 to 5 days, in the administration of antimicrobial after initial administration of a bismuth salt. Such preferred methods thus comprise the steps of:
a) administering to said subject from 50 to 5000 milligrams of a bismuth salt, per day, for from 3 to 56 days; and
b) administering to said subject a safe and effective amount of an antimicrobial, per day, for from 1 to 21 days;
wherein said step of administering an antimicrobial is commenced from 1 to 21 days after the commencement of said step of administering bismuth. Also preferably, a sample of gastric material is obtained from the stomach of the subject, and the infecting CLO cultured and tested for sensitivity to the antimicrobials useful herein (as described above) during the period of initial administration of bismuth but prior to administration of antimicrobial.

Suitable methods for detecting CLO in the upper gastrointestinal tract of human or other animal subjects include gram stains of gastric tissues (obtained, for example, by biopsy), serologic tests to detect the presence of antibodies to the organisms, tests of body fluids to detect the presence of metabolites of the organisms, silver or other specially stained tissue sections of tissues, and detection of urease enzyme in the stomach of the subject.

One preferred method of detection is the detection of urease enzyme (urea amidohydrolase) in the stomach of the human or lower animal having a gastrointestinal disorder. Such a detection step may include, for example, obtaining a sample of gastric fluid (e.g., from a gastric tube or vomitus) or of gastric mucosa (e.g., by biopsy) and analyzing the material for the presence of urease enzyme. One such method for the diagnosis of gastrointestinal disorder involves obtaining a sample of gastric mucosa and placing said sample into a composition which comprises:
a) urea, at a concentration of from about 10 to about 40 g/ℓ;
b) a bactericide, at a concentration of from about 1 to about 5 g/ℓ;
c) an indicator having a pKₐ of from about 6.5 to about 8.5, at an effective concentration; and
d) water;
wherein said composition has a pH of from about 5.0 to about 6.5 and said pH is at least one pH unit lower than the pKₐ of said indicator. Preferably, the composition contains a gelling agent, such as a non-nutritive agar, at a concentration of from about 5 to about 50 g/ℓ. Typically, the indicator is present at a concentration of from about 20 to about 100 mg/ℓ (As used herein, all concentrations are by weight of component per total volume of composition.) A change in the color of the composition indicates the presence of urease enzyme, and the presence of a gastrointestinal disorder.

The following non-limiting examples illustrate the kits of the present invention.

### EXAMPLE I

A human subject, suffering from atrophic gastritis, was treated using a kit of the present invention. Specifically, the subject was endoscoped and a biopsy was taken of the gastric mucosa of the subject. Analysis of the biopsy sample showed inflammation of the mucosa, and depletion of the protective mucous layer. The subject was then treated by administering a composition containing tripotassium dicitrato bismuthate, manufactured by Gist-Brocades, N.V. and sold under the name "DeNol". The composition, in tablet form (each tablet containing 120 mg of bismuth) was administered four times daily (for a total of 480 mg of bismuth administered per day) for 28 days. Amoxicillin tablets (each tablet contained 500 mg) was concurrently administered three times daily (for a total of 1500 mg antibiotic per day) for 21 days. The subject was endoscoped and biopsied again 28 days after treatment began, finding essentially normal, healed gastric mucosa. The subject remained asymptomatic, and another biopsy performed five months later also revealed normal gastric mucosa.

In the above example, bismuth citrate, bismuth tartrate, bismuth subcitrate and bismuth subsalicylate are substituted, respectively, for tripotassium dicitrato bismuthate, with substantially-similar results. Similarly, penicillin, doxycycline, erythromycin and ampicillin are substituted, respectively, for amoxycillin, with substantially-similar results.

### EXAMPLE II

A human subject, suffering from peptic ulcer disease, is treated using a kit of this invention. Specifically, a sample of vomitus is obtained from the individual and analyzed for the presence of urease. After detecting urease, the individual is then treated by administering 500 mg of bismuth subsalicylate, per day (two doses per day) for 28 days. After the fifth day (commencing on the sixth day) of bismuth treatment, the subject is also treated by administering 750 mg of nitofurantoin, per day, for 14 days. (Hence, the bismuth treatment continues for 9 days after the last day of antimicrobial treatment.) The subject is then endoscoped, revealing healing of the peptic ulcer lesion.

In the above example, nitrofurazone, metronidazole, and tinidazole are substituted, respectively, for nitrofurantoin, with substantially-similar results.

### EXAMPLE III

A human subject, suffering from non-ulcer dyspepsia, is treated using a kit of this invention. Specifically, a biopsy of gastric mucosa is taken from the stomach of the subject. The sample is then placed in 0.5 mℓ of an aqueous gel of a composition having the following composition:

| Component | Quantity grams | Final Concentration |
|---|---|---|
| urea | 3.000 | 30 g/l |
| phenol red* | 0.008 | 80 mg/l |
| methyl hydroxy benzoate | 0.200 | 2 g/l |
| bacteriological agar | 1.500 | 15 g/l |
| citric acid | 0.040 | 400 mg/l |
| sodium phosphate | 0.080 | 800 mg/l |

| | | |
|---|---|---|
| *: phenol sulfonphthalein indicator, having pKₐ = 7.9, exhibiting a yellow color in undissociated state (below pH 6.4) and red color in dissociated state (above pH 8.2) (The components, except urea, are dissolved in 100 milliliters of water, heated to approximately 65°C, and stirred until the solution is clear. The composition is allowed to cool to below approximately 45°C and the urea is added. Upon cooling to ambient temperature, a gel is formed having a pH of 6.0 and a deep yellow color.) | | |

After the biopsy sample is inserted into the composition, the composition color changes from yellow to red over a period of approximately fifteen minutes, indicating the presence of urea in the biopsy sample and presence of CLO in the stomach of the subject. The subject is then treated by administering 500 mg of bismuth, as bismuth subsalicylate, per day, for 10 days. On the tenth day of bismuth treatment, the subject is also administered 5000 mg of penicillin, by intraveneous injection. Five days later the subject is endoscoped, revealing normal gastric mucosa, and a biopsy sample obtained. The sample is then inserted into 0.5 mℓ of a test composition comprised as above, and the color of the composition remained unchanged after 24 hours, indicating lack of CLO infection.

## Claims

1. Kit-of-parts for use in the treatment of a human or other animal subject having an infectious gastrointestinal disorder, the kit-of-parts comprising from 3 to 56 daily dosages of a bismuth compound or bismuth-containing composition containing from 50 to 5000 mg of bismuth per dosage and from 1 to 21 daily dosages of an antimicrobial or antimicrobial-containing composition containing from 100 to 10,000 mg of antimicrobial per dosage and wherein the antimicrobial is selected from gentamicin, neomycin, kanamycin, streptomycin, erythromycin, clindamycin, rifampin, penicillin G, penicillin V, ampicillin, amoxycillin, bactracin, polymyxin, tetracycline, chlortetracycline, oxytetracycline, doxycyline, cephalexin, cephalothin, chloramphenicol, sulfonamides, nitrofurazone, nitrofurantoin, furozolidone, metronidazole, tinidazole, nimorazole, and mixtures thereof.

2. Kit-of-parts according to Claim 1, wherein the daily dosages of bismuth compound or bismuth-containing composition containing from 500 to 1500 mg of bismuth per dosage.

3. Kit-of-parts according to Claim 1 or 2 for the treatment of a non-ulcerative gastrointestinal disorder wherein the kit-of-parts comprises from 3 to 21 daily dosages of said bismuth compound or bismuth-containing composition.

4. Kit-of-parts according to Claim 1 or 2 for the treatment of a peptic ulcer disease wherein the kit-of-parts comprises from 14 to 56 daily dosages of said bismuth compound or bismuth-containing composition.

5. Kit-of-parts according to any one of Claims 1 to 4 wherein said bismuth compound is selected from bismuth aluminate, bismuth subcarbonate, bismuth subcitrate, bismuth citrate, tripotassium dicitrato bismuthate, bismuth subgalate, bismuth subnitrate, bismuth tartrate, bismuth subsalicylate, and mixtures therof.

6. Kit-of-parts according to any one of Claims 1 to 5 wherein said bismuth compound is tripotassium dicitrato bismuthate.

7. Kit-of-parts according to any one of Claims 1 to 5 wherein said bismuth compound is bismuth subsalicylate.

## Patentansprüche

1. Kit-of-parts zur Verwendung bei der Behandlung eines Menschen oder eines anderen Tieres mit infektiöser Störung des Gastrointestinaltraktes, welcher Kit-of-parts 3 bis 56 tägliche Dosen einer Bismutverbindung oder einer Bismut enthaltenden Zusammensetzung mit einem Gehalt an 50 bis 5000 mg Bismut je Dosis, und 1 bis 21 tägliche Dosen eines antimikrobiellen Mittels oder einer ein antimikrobielles Mittel enthaltenden Zusammensetzung mit einem Gehalt an 100 bis 10.000 mg antimikrobiellem Mittel je Dosis umfaßt, und worin das antimikrobielle Mittel unter Gentamicin, Neomycin, Kanamycin, Streptomycin, Erythromycin, Clindamycin, Rifampin, Penicillin G, Penicillin V, Ampicillin, Amoxycillin, Bacitracin, Polymyxin, Tetracyclin, Chlortetracyclin, Oxytetracyclin, Doxycyclin, Cephalexin, Cephalothin, Chloramphenicol, Sulfonamiden, Nitrofurazon, Nitrofurantoin, Furozolidon, Metronidazol, Tinidazol, Nimorazol und deren Gemischen ausgewählt wird.

2. Kit-of-parts nach Anspruch 1, worin die tägliche Dosis der Bismutverbindung oder der Bismut enthaltenden Zusammensetzung 500 bis 1500 mg Bismut je Dosis enthält.

3. Kit-of-parts nach Anspruch 1 oder 2 zur Behandlung einer nicht-ulcerierenden gastrointestinalen Störung, worin der Kit-of-parts 3 bis 21 tägliche Dosen dieser Bismutverbindung oder Bismut enthaltenden Zusammensetzung umfaßt.

4. Kit-of-parts nach Anspruch 1 oder 2 zur Behandlung einer Erkrankung vom Typus eines Ulcus pepticum, worin der Kit-of-parts 14 bis 56 tägliche Dosen dieser Bismutverbindung oder Bismut enthaltenden Zusammensetzung umfaßt.

5. Kit-of-parts nach einem der Ansprüche 1 bis 4, worin die Bismutverbindung unter Bismutaluminat, Bismutsubcarbonat, Bismutsubcitrat, Bismutcitrat, Trikaliumdicitratobismutat, Bismutsubgalat, Bismutsubnitrat, Bismuttartrat, Bismutsubsalicylat und deren Gemischen ausgewählt ist.

6. Kit-of-parts nach einem der Anspruche 1 bis 5, worin diese Bismutverbindung Trikaliumdicitratobismutat ist.

7. Kit-of-parts nach einem der Anspruche 1 bis 5, worin diese Bismutverbindung Bismutsubsalicylat ist.

## Revendications

1. Ensemble contenant des éléments à utiliser dans le traitement d'un sujet humain ou animal ayant un trouble gastro-intestinal infectieux, l'ensemble contenant des éléments comprenant de 3 à 56 doses journalières d'un composé de bismuth ou d'une composition contenant du bismuth, renfermant de 50 à 5000 mg de bismuth par dose et de 1 à 21 doses journalières d'un antimicrobien ou d'une composition contenant un antimicrobien, renfermant de 100 à 10.000 mg d'antimicrobien par dose, et l'antimicrobien étant choisi parmi la gentamicine, la néomycine, la kanamycine, la streptomycine, l'érythromycine, la clindamycine, la rifampine, la pénicilline G, la pénicilline V, l'ampicilline, l'amoxycilline, la bactracine, la polymyxine, la tétracycline, la chlortétracycline, l'oxytétracycline, la doxycyline, la céphalexine, la céphalothine, le chloramphénicol, les sulfonamides, la nitrofurazone, la nitrofurantoïne, la furozolidone, le métronidazole, le tinidazole, le nimorazole, et leurs mélanges.

2. Ensemble contenant des éléments selon la revendication 1, dans lequel les doses journalières du composé de bismuth ou de la composition contenant du bismuth renferment de 500 à 1500 mg de bismuth par dose.

3. Ensemble contenant des éléments selon la revendication 1 ou 2, pour le traitement d'un trouble gastro-intestinal non ulcératif, dans lequel l'ensemble contenant des éléments comprend de 3 à 21 doses journalières dudit composé de bismuth ou de ladite composition contenant du bismuth.

4. Ensemble contenant des éléments selon la revendication 1 ou 2, pour le traitement d'un ulcère peptique, dans lequel l'ensemble contenant des éléments comprend de 14 à 56 doses journalières dudit composé de bismuth ou de ladite composition contenant du bismuth.

5. Ensemble contenant des éléments selon l'une quelconque des revendications 1 à 4, dans lequel ledit composé de bismuth est choisi parmi l'aluminate de bismuth, le sous-carbonate de bismuth, le sous-citrate de bismuth, le citrate de bismuth, le dicitrato-bismuthate tripotassique, le sous-galate de bismuth, le sous-nitrate de bismuth, le tartrate de bismuth, le sous-salicylate de bismuth, et leurs mélanges.

6. Ensemble contenant des éléments selon l'une quelconque des revendications 1 à 5, dans lequel ledit composé de bismuth est le dicitrato-bismuthate tripotassique.

7. Ensemble contenant des éléments selon l'une quelconque des revendications 1 à 5, dans lequel ledit composé de bismuth est le sous-salicylate de bismuth.
